# EUROPEAN PATENT APPLICATION

(11) **EP 3 088 026 A1**
(43) Date of publication of application: **02.11.2016**
(21) Application number: 15165391.2
(22) Date of filing: 28.04.2015
(51) Int. Cl.: A61M 5/24

(54) **DATA COLLECTION DEVICE FOR A MEDICAMENT DELIVERY DEVICE**

(71) Applicant: Sanofi-Aventis Deutschland GmbH, 65929 Frankfurt am Main (DE)
(72) Inventor: The designation of the inventor has not yet been filed

(57) **Abstract**

A data collection device comprises a body and a mating arrangement for releasable attachment to a medicament delivery device. The mating arrangement includes at least one engaging member configured to engage with a cooperating formation on the delivery device and a cover portion that overlies at least part of said engaging member. At least part of the cover portion is a non-opaque section, such as a window. The non-opaque section may have, or include a sub-section having, some optical power to provide a magnified view. The non-opaque section allows a user to view said part of the engaging member and the cooperating formation, facilitating positioning and alignment of the data collection device and allowing visual verification that engagement has occurred. The data collection device may comprise an arrangement to detect movement of a dosage programming element of the delivery device, from which a medicament dosage amount programmed into the delivery device and/or delivered may be determined.

## Description

### Field

This disclosure relates to a data collection device to supplement a medical device configured to deliver a medicament.

### Background

A variety of diseases exists that require regular treatment by injection of a medicament. Such injections can be administered either by medical personnel or by patients themselves. As an example, type-1 and type-2 diabetes can be treated by patients themselves by injection of insulin doses, for example once or several times per day. A pre-filled disposable insulin pen or, alternatively, a re-usable pen can be used as an injection device. A re-usable pen allows replacement of an empty medicament cartridge by a new one. Either type of pen may come with a set of one-way needles, so that a new needle may be mounted onto the pen for each injection. The insulin dose to be injected can be manually selected at the insulin pen, for example by turning a dosage knob and observing the actual dose from a dose window or display of the insulin pen. The dose is then injected by inserting the needle into the patient's skin and pressing an injection button of the insulin pen.

To be able to monitor injections, for instance to prevent false handling of the injection device, to keep track of the doses already applied or to gather information for determining adjustments to a patient's treatment regime, it is desirable to measure information related to a condition and/or use of the injection device. For example, recordal of one or more of the type of medicament, administered dosage amount and the timing of an injection may be required, in a manner that is reliable and accurate. WO 2011/117212 discloses a supplementary device comprising a mating unit for releasably attachment to an injection device. The device includes a camera and is configured to perform optical character recognition (OCR) on images of a dosage window of the injection pen, to determine a dose of medicament that has been dialled into the injection device.

The proper attachment of such a supplementary device to an injection device is important for various reasons, for example reliable data gathering, user comfort, ease of use, etc. On one hand, the attachment has to be reliable so that the supplementary device does not fall off during use. On the other hand the act of attachment should be easy to perform and/or be user friendly. For example, attaching the supplementary device should not require a high force, not be complicated and should not take a long time. Furthermore, the position of the device should remain the same during use to provide reliable data capturing, in particular when a camera is used. Furthermore, finding the right position in terms of aligning the supplementary device with the injection device is also important, for example, to ensure that cooperating features are close to each other and/or can engage. This is particularly important when a camera is used in the supplementary device for data collection, so that the camera is correctly positioned and aligned to capture an image of a particular part of the injection device.

The present invention aims at providing an improved supplementary device that addresses at least one of the problems mentioned above.

### Summary

According to a first aspect, there is provided a data collection device including a body and a mating arrangement configured to releasably attach the body to a medicament delivery device, the mating arrangement having at least one engaging member configured to engage with a cooperating formation on the medicament delivery device, and a cover portion that overlies at least part of said engaging member, at least a part of the cover portion being a non-opaque section through which said at least part of said engaging member is viewable.

This aspect also provides a medicament delivery system including a medicament delivery device and such a data collection device.

The provision of the non-opaque section can allow a user to verify visually that the engaging member is properly engaged with the cooperating formation on the medicament delivery device and, therefore, that the data collection device is mounted on the medicament delivery device with correct positioning and alignment.

The mating arrangement may include one or more resilient members arranged to bias the at least one engaging member into engagement with the cooperating formation when the data collection device is attached to the medicament delivery device. In one example, the at least one engaging member is a first engaging arm, the mating arrangement further includes a second engaging arm, and the one or more resilient members bias free ends of the first and second engaging arms towards each other. Optionally, an actuating section may be provided to urge the at least one engaging member out of engagement with the cooperating formation.

The at least one engaging member is rotatable between an engaged position and a detached position.

The mating arrangement may include a collar which is configured to receive a medicament delivery device. Such a collar may be configured to be pivoted between a first position in which the medicament delivery device is slidably receivable through the collar and a secured position in which the at least one engaging member is engageable with the cooperating formation to attach the data collection device to the medicament delivery device.

The non-opaque section may be, or may include, a window provided in said cover portion. Optionally, the window may have an optical power to provide a magnified view of said at least part of said engaging member. In one example, the window is made from glass.

In some embodiments, the non-opaque section includes a sub-section having an optical power to provide a magnified view of said at least part of said engaging member. In one example, the sub-section is made from plastic.

The data collection device may include a camera unit configured to capture images of a display on the medicament dosage indicator of the medicament delivery device when the data collection device is attached to the medicament delivery device. Optionally, a processing arrangement may be provided in the data collection device that is configured to determine a medicament dosage amount programmed into the medicament delivery device based on at least one image of the medicament dosage indicator captured by said camera unit. Optionally, the processing arrangement may be configured to control a unit, for example a wireless unit, configured to transmit and/or receive data and/or information to/from another device. Wireless transmission may for instance be based on radio transmission or optical transmission. The data and/or information transmitted may include a captured image or information determined from an image, for example a medicament dosage amount.

Additionally, or alternatively, the data collection device may include a sensing arrangement comprising one or more sensors arranged to detect movement of a dosage programming element of the medicament delivery device. In such a device, a processing arrangement may be configured to determine a medicament dosage amount programmed into the medicament delivery device based on movement detected by said sensing arrangement. Optionally, the processing arrangement may be configured to control a unit, for example a wireless unit, configured to transmit and/or receive data and/or information to/from another device. Wireless transmission may for instance be based on radio transmission or optical transmission. The data and/or information transmitted may include data from the sensing arrangement or information determined from movement detected by said sensing arrangement, for example a medicament dosage amount.

### Brief Description of the Drawings

Embodiments of the invention will now be described, by way of example only, with reference to the accompanying drawings, in which:
Figure 1 is an exploded view of an example medicament delivery device;
Figure 2 is a perspective view of a part of the medicament delivery device shown in Figure 2;
Figure 3 is a perspective view of a data collection device according to a first embodiment when releasably attached to the medicament delivery device of Figure 1;
Figure 4 is a block diagram of the data collection device of Figure 3 in a state when attached to the medicament delivery device of Figure 1;
Figure 5 is a flowchart of an example data collection method that may be performed by the data collection device of Figure 3;
Figures 6, 7 and 8 depict stages in a process of attaching the data collection device of Figure 3 to the medicament delivery device of Figure 1;
Figure 9 is a perspective view of the data collection device of Figure 3, shown with a wing portion omitted;
Figure 10 is a cross-sectional view of the data collection device of Figure 3 shown with the wing portion omitted, before attachment to the medicament delivery device of Figure 1;
Figure 11 is a cross-sectional view of the data collection device of Figure 3 shown with the wing portion omitted, when releasably attached to the medicament delivery device of Figure 1;
Figure 12 is a cross-sectional view of the data collection device of Figure 3 shown with the wing portion omitted, when a mating arrangement of the data collection device is disengaged from the medicament delivery device of Figure 1;
Figure 13 depicts a part of the mating arrangement of the data collection device of Figure 3;
Figure 14 depicts another part of the mating arrangement of the data collection device of Figure 3;
Figure 15 is a cross-sectional view of the part of the mating arrangement of shown in

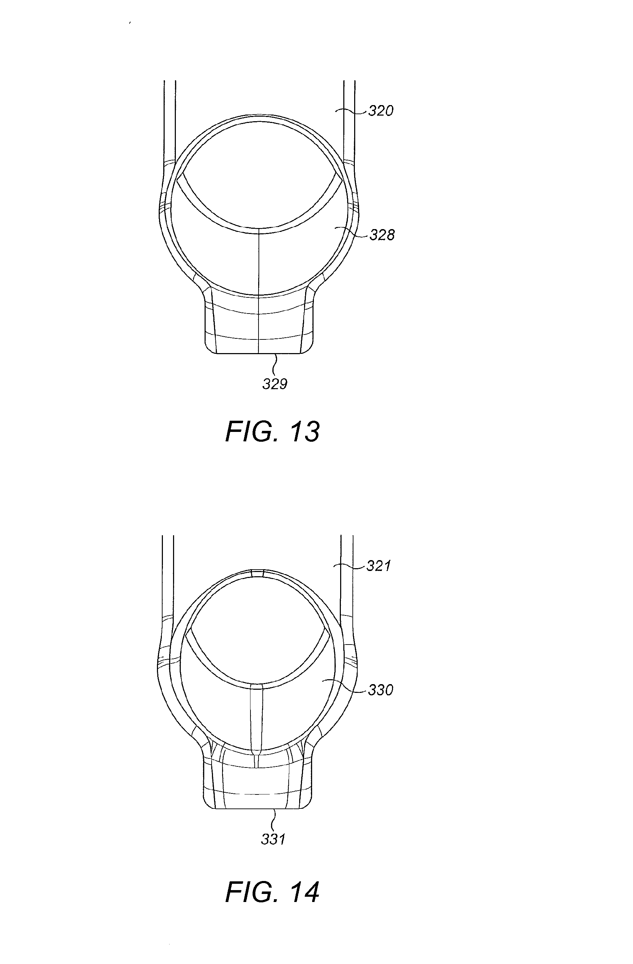

Figure 14 being received on a pivot axle; and

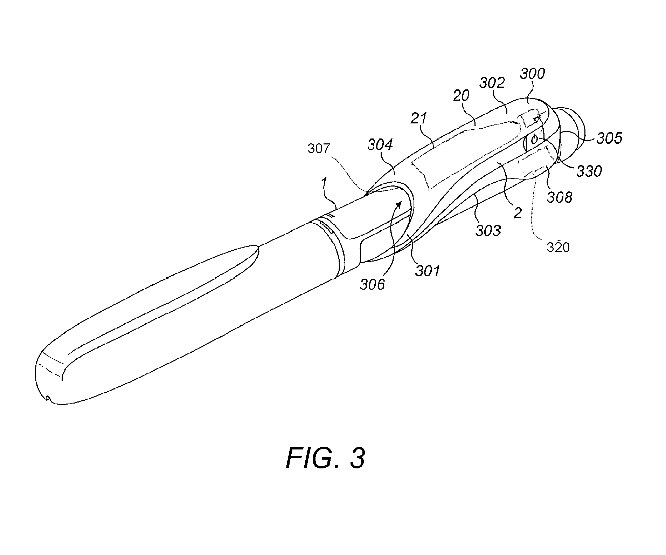

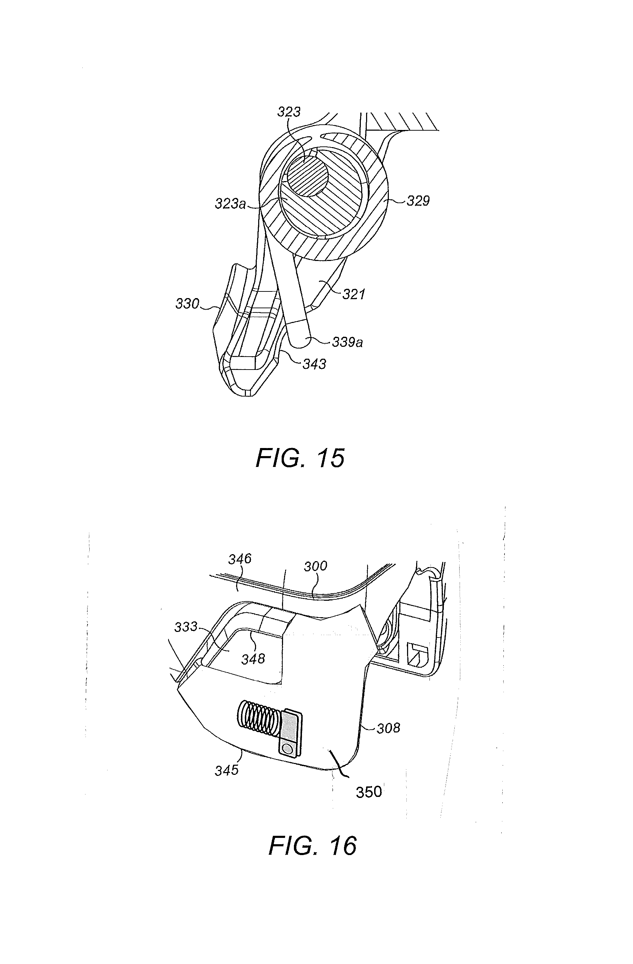

Figure 16 is a perspective view of a part of the data collection device of Figure 3.

### Detailed Description

In the following, embodiments of the present invention will be described with reference to an insulin injection device. The present invention is however not limited to such application and may equally well be deployed with injection devices that eject other medicaments, or with other types of medicament delivery devices.

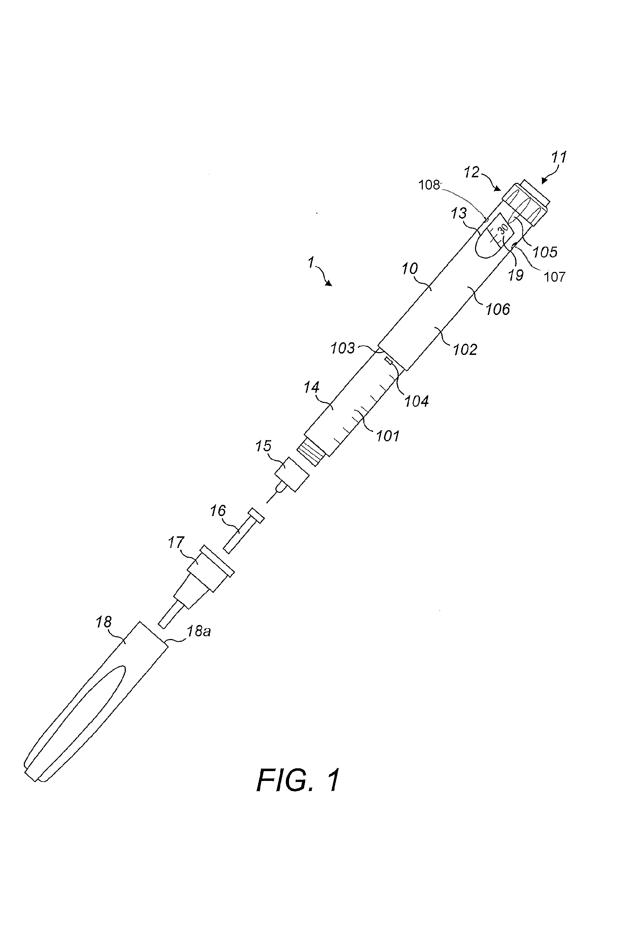

Figure 1 is an exploded view of an example medicament delivery device 1, which may for instance represent Sanofi's Solostar® insulin injection pen.

In this particular example, the medicament delivery device 1 is a pre-filled, disposable injection pen having a housing 10 and an insulin container 14, to which a needle 15 can be affixed. The needle is protected by an inner needle cap 16 and either an outer needle cap 17 or other cap 18.

An insulin dose to be ejected from medicament delivery device 1 can be programmed, or 'dialled in' by turning a dosage knob 12, and a currently programmed dose is displayed in a dosage window 13, for instance in multiples of units. For example, where the medicament delivery device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1, for example using an electronic display. It will be understood that the term dosage window relates to the section of the medicament delivery device through or on which the selected dosage is visible.

The dosage window 13 may be in the form of an aperture in the housing 10, which permits a user to view a limited portion of a number sleeve 70 that is configured to move when the dosage knob 12 is turned, to provide a visual indication of a currently programmed dose. The dosage knob 12 is rotated on a helical path with respect to the housing 10 when turned during programming.

Optionally, turning the dosage knob 12 causes a mechanical click sound to provide acoustical feedback to a user. In this example, the numbers displayed in the dosage window 13 are printed on a sleeve that is contained in housing 10 and mechanically interacts with a piston in insulin container 14. When the injection button 11 is pushed, the insulin dose displayed in display 13 will be ejected from the medicament delivery device 1. When the needle 15 of the medicament delivery device 1 remains for a certain time in the skin portion after the injection button 11 is pushed, a high percentage of the dose is actually injected into the patient's body. Ejection of the insulin dose also causes a mechanical click sound, which is however different from the sounds produced when turning dosage knob 12.

The medicament delivery device 1 may be used for several injection processes until either insulin container 14 is empty or the expiration date of the medicament delivery device 1, for example 28 days after the first use, is reached.

Before using the medicament delivery device 1 for the first time, it may be necessary to perform a so-called "prime shot" to remove air from insulin container 14 and needle 15, for instance by selecting two units of insulin and pressing injection button 11 while holding the medicament delivery device 1 with the needle 15 upwards.

For simplicity of presentation, in the following, it will be assumed that the ejected doses substantially correspond to the injected doses, so that, for instance when a dosage amount is programmed into the medicament delivery device 1, this dose equals the dosage amount that is injected into the patient. Nevertheless, differences (e.g. losses) between the programmed medicament dosage amount, ejected doses and the injected doses may of course be taken into account.

The housing 10 of the medicament delivery device 1 comprises a front section 101 and a rear section 102. The needle 15 is affixed to a front end of the front section 101 and the dosage knob 12 extends from a rear end of the rear section 102.

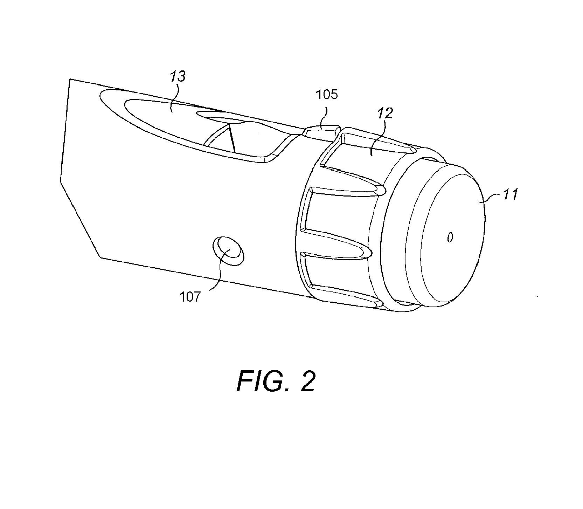

As shown in Figures 1 and 2, a rib 105 protrudes from an outer surface 106 of the medicament delivery device 1. The rib 105 acts as an alignment element for locating a supplementary data collection device in a specific position relative to the outer surface 106 of the medicament delivery device 1. In addition, first and second indents 107, 108 are formed in the outer surface 106 of the medicament delivery device 1. The indents 107, 108 may have chamfered sides. Each indent 107, 108 may be a notch.

Figure 3 is a schematic illustration of a data collection device 2 releasably attached to the medicament delivery device 1 of Figure 1. The data collection device 2 has a housing 20 with a mating arrangement configured to engage the housing 10 of the medicament delivery device 1 so that the data collection device 2 is attached to the housing 10 of the medicament delivery device 1, but is nevertheless removable from the medicament delivery device 1, for instance when the insulin container 14 is empty and the medicament delivery device 1 has to be replaced.

In this particular example, the housing 20 of the data collection device 2 has a body 300 and a collar 301. The body 300 is elongate and a display unit 21 is disposed on an upper side 302 of the body 300. The collar 301 extends from a lower side 303 of the body 300. The body 300 has a front end 304 and a rear end 305. The collar 301 extends from the front end 304. The collar 301 extends from the body 300 at an acute angle to the longitudinal axis of the elongate body 300.

The collar 301 has an aperture 306 formed therethrough. The collar 301 is configured to receive the medicament delivery device 1 through the aperture 306. A channel 307 is formed in the lower side 303 of the body 300, extending between the front end 304 and the rear end 305 of the body 300.

Two wings 308, acting as protective walls or cover portions, extend downwardly from the lower side 303 of the body 300. The wings 308 are spaced from each other and distend from either side of the channel 307. Therefore, the medicament delivery device 1 is receivable in the channel 307 between the wings 308. The wings 308 are disposed at the rear end 305 of the body 300, at an opposite end of the body 300 to the collar 301.

As will be described later, at least part of one or both of the wings 308 is non-opaque, so that part of the mating arrangement of the data collection device 2 can be viewed therethrough. In this particular example, a non-opaque section 350 formed of a non-opaque material is located in the wing 308. However, in another embodiment, the wing 308 itself may be a non-opaque section, formed of a non-opaque material. In yet another embodiment, the non-opaque section may be a window formed of a non-opaque material in the wing 308. Examples of suitable non-opaque materials include a non-opaque polycarbonate material, polyvinylchroide (PVC), polymethylacrylate, other non-opaque plastics materials or glass.

The provision of a non-opaque section 350 can facilitate correct alignment and/or positioning of the data collection device 2, by allowing a user to view part of the mating arrangement and part of the housing 10 of the medicament delivery device 1 to assist in positioning the data collection device 2 relative to the medicament delivery device 1 and/or to verify visually that engagement between the part of the mating arrangement of the data collection device 2 with one or both of the first and second indents 107, 108 has occurred. Through the non-opaque section 350 the user can also see a misalignment of the data collection device 2 by way of seeing the relative position between part of the mating arrangement and one or both first and second indents 107, 108. The user could then directly conclude in which direction the two devices should be moved relative to each other in order to achieve engagement between the part of the mating arrangement of the data collection device 2 with one or both of the first and second indents 107, 108.

To provide further assistance to the user, the non-opaque section 350 may be configured to have an optical power to provide a magnified view of the part of the mating arrangement. In general providing a magnified view of the part of the mating arrangement improves viewing the part of the mating arrangement because the part of the mating arrangement appears bigger in the field of view of the user. Therefore viewing small structures is improved, as for example an engagement arm or part thereof, or a protuberance at an engagement arm. A further benefit is that the field of view of the user is focussed to the part of the mating arrangement so he/she can more easily find the area of interest.

For example, in one embodiment, the non-opaque section 350 may be, or may include, a magnifying window. For example, the non-opaque section 350 may include a magnifying window, made from suitable transparent plastic materials or from glass. A glass window may be capable of achieving a given level of magnification with a more compact design when compared to windows made from materials such as polycarbonate, polymethacrylate, hard PVC (polyvinylchloride) or other suitable plastic materials.

In another embodiment, the non-opaque section includes a subsection that has an optical power to provide a magnified view of the part of the mating arrangement. Such a sub-section may be formed from plastics material as it may be easier to manufacture a non-opaque section that includes a sub-section from plastics when compared with glass.

The collar 301 and channel 307 form part of an alignment arrangement or alignment unit to locate the data collection device 2 in a specific position relative to the outside surface 106 of the medicament delivery device 1. The alignment unit forms part of the mating arrangement configured to embrace the housing 10 of medicament delivery device 1 to maintain the data collection device 2 in a specific position on the medicament delivery device 1.

The mating arrangement also includes an engaging unit or arrangement configured to releasably mount the body to the medicament delivery device 1. The collar 301 also forms part of the engaging unit.

The data collection device 2 may include optical and acoustical sensors for gathering information from the medicament delivery device 1. At least a part of this information, for instance a selected dose and, optionally, a unit of this dose, is displayed via display unit 21 of data collection device 2, since the dosage window 13 of the medicament delivery device 1 is obstructed from view by the data collection device 2.

The data collection device 2 includes one or more input transducers, illustrated schematically as a button 330. These input transducers allow a user to turn on/off the data collection device 2 to trigger actions. Such actions may include, for example, causing establishment of a connection to or a pairing with another device and/or transmission of information from the data collection device 2 to another device. The button 330 may also, or alternatively, be used to allow the user to confirm information or an instruction.

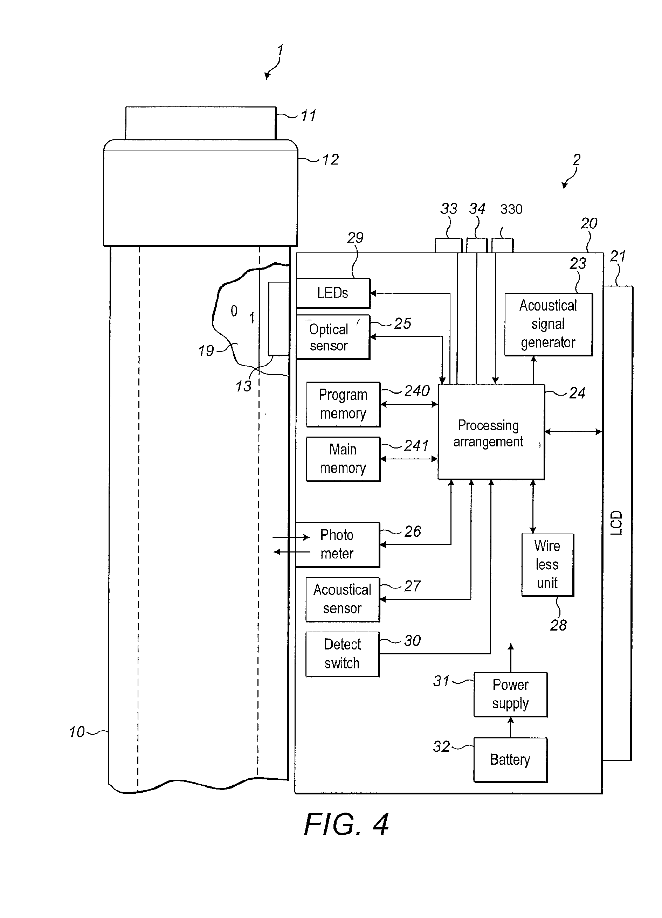

Figure 4 is a block diagram of the data collection device 2 of Figures 2 and 3 when attached to the medicament delivery device 1 of Figure 1.

The data collection device includes a processing arrangement 24, which may for instance be a microprocessor, a Digital Signal Processor (DSP), Application Specific Integrated Circuit (ASIC), Field Programmable Gate Array (FPGA) or the like. Processor 24 executes program code (e.g. software or firmware) stored in a program memory 240, and uses a main memory 241, for instance to store intermediate results. Main memory 241 may also be used to store a logbook on performed ejections/injections. Program memory 240 may for instance be a Read-Only Memory (ROM), and main memory may for instance be a Random Access Memory (RAM).

In an example embodiment, processing 24 interacts with the button 330, via which data collection device 2 may for instance be turned on and off. A second button 33 is a communications button. The second button may be used to trigger establishment of a connection to another device, or to trigger a transmission of information to another device. A third button 34 is a confirm or OK button. The third button 34 can be used to acknowledge information presented to a user of the data collection device 2. The buttons 33, 34, 330 may be any suitable form of user input transducers, for instance mechanical switches, capacitive sensors or other touch sensors.

The processing arrangement 24 controls the display unit 21, which, in this example, is a Liquid Crystal Display (LCD). The display unit 21 is used to display information to a user of the medicament delivery device 2, for instance on present settings of the medicament delivery device 1, or on a next injection to be given. In some embodiments, the display unit 21 may also be embodied as a touch-screen display to receive user input.

In this example, the processing arrangement 24 also controls an optical sensor 25, embodied as a camera and Optical Character Recognition (OCR) unit, that is capable of capturing images of the dosage window 13, in which a currently selected dosage amount is displayed. The OCR unit is capable of recognizing characters, such as numbers, from the captured images and arranged to provide this information to the processing arrangement 24.

Alternatively, the optical sensor 25 may include a camera without an OCR facility. The camera captures images of the dosage window 13 and provides information on the captured images to processing arrangement 24. Then processing arrangement may optionally perform pattern recognition or OCR on the captured images to determine a currently selected dosage amount, or simply forward the images to another device for analysis, via a wired or wireless connection, for example using wireless unit 28.

Optionally, the optical sensor 25 may include a lens, such as an aspheric lens, to provide magnification. In ones example, such a lens is used to provide a magnification of more than 3:1 .

The processing arrangement 24 may also present some or all of images captured by a camera of the optical sensor 25 using the display 21, so that a user can view the currently selected dosage amount displayed in the dosage window 13.

The processing arrangement 24 also controls light-sources such as light emitting diodes (LEDs) 29 to illuminate the dosage window 13. A diffuser may be used in front of the light-sources, for instance a diffuser made from a piece of acrylic glass.

The data collection device 2 may also include a photometer 26, that is configured to determine an optical property of the housing 10 of the medicament delivery device 1, for example a colour or a shading. Information on this colour is then provided to processing arrangement 24, which may then determine the type of the medicament delivery device 1 or the type of insulin contained in the medicament delivery device 1, for example, a SoloStar Lantus® pen may be indicated by a particular purple colour, while a SoloStar Apidra® pen may be indicated by a particular blue colour. Alternatively, a camera unit may be used instead of photometer 26, and an image of the housing, sleeve or insulin container may then be provided to processing arrangement 24 to determine the colour of the housing, sleeve or insulin container by way of image processing. Further, one or more light sources may be provided to improve reading of photometer 26. The light source may provide light of a certain wavelength or spectrum to improve colour detection by photometer 26. In an example embodiment, instead of or in addition to photometer 26, a camera unit may be deployed to detect a code (for instance a bar code, which may for instance be a one- or two-dimensional bar code) related to the medicament delivery device 1 and/or the medicament contained therein. This code may for instance be located on the housing 10 or on a medicament container 14 to name but a few examples. This code may for instance indicate a type of the medicament delivery device and/or the medicament, and/or further properties, for instance, an expiration date.

An acoustic sensor 27 may be provided, configured to sense sounds produced by medicament device 1. Such sounds may for instance occur when a dose is dialled by turning dosage knob 12 and/or when a dose is ejected/injected by pressing injection button 11, and/or when a prime shot is performed. These actions are mechanically similar but nevertheless sound different from one another. Either the acoustic sensor 27 and/or processing arrangement 24 may be configured to differentiate these different sounds, for instance to be able to recognize that an injection has taken place (rather than a prime shot only).

An acoustical signal generator 23 may be provided, configured to produce acoustical signals as feedback to the user. For example, an acoustical signal may be launched by acoustical signal generator 23 as a reminder for the next dose to be injected or as a warning signal, for instance in case of misuse. Acoustical signal generator may for instance be embodied as a buzzer or loudspeaker. In addition to or as an alternative to acoustical signal generator 23, also a haptic signal generator (not shown) may be used to provide haptic feedback, for instance by way of vibration.

The wireless unit 28 is configured to transmit and/or receive information to/from another device in a wireless fashion. Such transmission may for instance be based on radio transmission or optical transmission. In some embodiments, the wireless unit 28 is a Bluetooth transceiver. Alternatively, wireless unit 28 may be substituted or complemented by a wired unit configured to transmit and/or receive information to/from another device in a wire-bound fashion, for instance via a cable or fibre connection.

A pen detection switch 30 may be provided, which is operable to detect whether the medicament delivery device 1 is present, that is, whether the data collection device 2 is attached to the medicament delivery device 1.

A battery 32 powers the processing arrangement 24 and other components of the data collection device 2 by way of a power supply 31.

The data collection device 2 is thus capable of determining information related to a condition and/or use of the medicament delivery device 1. This information may be presented on the display 21. The information may be either processed by the data collection device 2 itself, or may at least partially be provided to another device, such as a blood glucose monitoring system or a computing device.

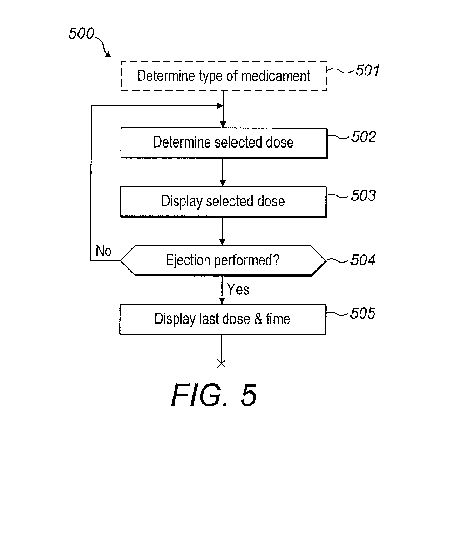

Figure 5 is a flowchart of a method 500 that may be performed by the processing arrangement 24 of the data collection device 2.

The method 500 may begin when the data collection device 2 is turned on or is otherwise activated. In a step 501, a type of medicament, for example insulin, provided by the injection device is determined, for instance based on colour recognition or based on recognition of a code printed on injection device or a component thereof as already described above. Detection of the type of medicament may not be necessary if a patient always takes the same type of medicament and only uses an injection device with this single type of medicament. Furthermore, determination of the type of medicament may be ensured otherwise. For example, the mating arrangement of the data collection device 2 may be configured so that the data collection device 2 is only useable with one specific type of medicament delivery device 1, for example by providing the first and second indents 107, 108 at particular positions and/or with shapes specific to a particular type of medicament delivery device 1, which may then only provide this single type of medicament.

In a step 502, a currently selected dose is determined, for instance by OCR of information shown the dosage window 13 as described above. This information is then presented on the display 21 in a step 503, so that it may be viewed by a user.

In a step 504, it is checked if an ejection has taken place, for instance by sound recognition as described above. Therein, a prime shot may be differentiated from an actual injection (into a creature) either based on respectively different sounds produced by the injection device and/or based on the ejected dose. For example, ejection of a dose that is less than a pre-defined amount of units, such as 3 or 4 units, may be considered to be to a prime shot, whereas an ejection of a larger dose may be considered to be an administered injection.

If an ejection has taken place, the determined data, that is, the selected dose and - if applicable - the type of medicament, is stored in the main memory 241, from where it may later be transmitted to another device, for instance a blood glucose monitoring system. If a differentiation has been made concerning the nature of the ejection, for instance if the ejection was performed as a prime shot or as an actual injection, this information may also be stored in the main memory 241, and possibly later transmitted. In the case of an injection having been performed, at step 505 the determined medicament dosage amount is shown on the display 21. Also displayed is a time since the last injection which, immediately after injection, is 0 or 1 minute. The time since last dose may be displayed intermittently. For instance, it may be displayed alternately with the name or other identification of the medicament that was injected.

If ejection was not performed at step 504, steps 502 and 503 are repeated.

After display of the delivered dose and time data, the method 500 ends.

When a user/patient gets a new medicament delivery device 1, the user attaches the data collection device 2 to the medicament delivery device 2 by way of the mating arrangement. The correct alignment of the data collection device 2 on the medicament delivery device 1 ensures that the optical sensor 25 is correctly aligned with the dosage window 13. Correct alignment facilitates reliable readings of the programmed medicament dosage amount. By ensuring that the alignment between the data collection device 2 and the medicament delivery device 1 is correct when in use may permit the use of a simpler design for the optical sensor 25 and, where OCR is used, simpler processing, because it might not be necessary to accommodate variations in alignment.

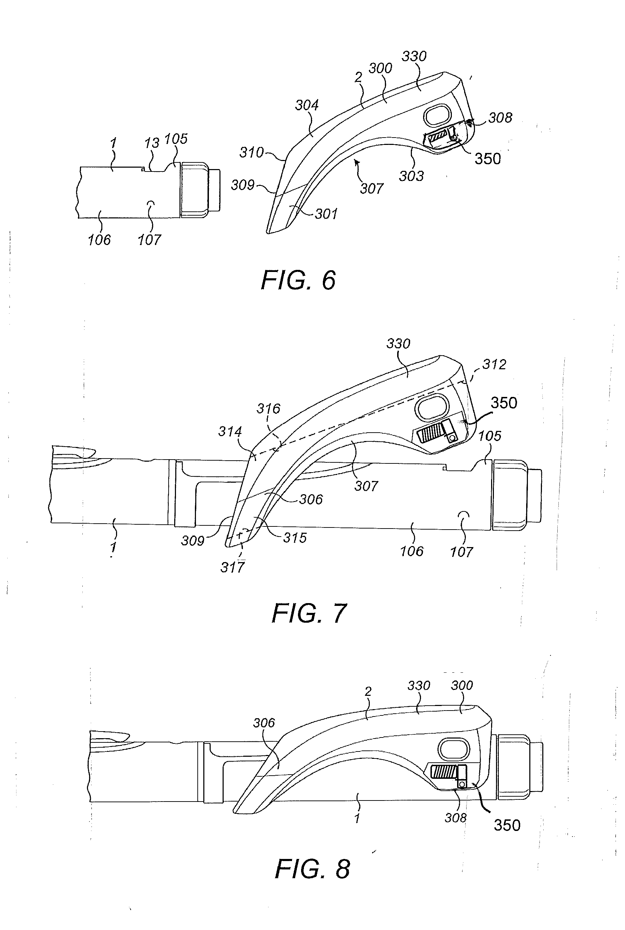

In Figure 6, the data collection device 2 is shown prior to mounting on the medicament delivery device 1. In Figure 6, the data collection device 2 is shown orientated relative to the rear section 102 of the housing 10 of the medicament device 1 so that the rear section 102 is receivable through the aperture 306 formed in the collar 301.

The aperture 306 has a front opening 309. The front opening 309 is formed in a front face 310 of the housing 20. The front face 310 may be planar. The edge of the front opening 309 is defined on a plane extending at an angle to the longitudinal axis of the elongate body 300. The front opening 309 has an elliptical shape. The width of the front opening 309 at its minor axis or conjugate diameter corresponds to or is slightly greater than the diameter of the rear section 102 of the medicament delivery device 1. The width of the front opening 309 at its major axis or transverse diameter is greater than the diameter of the rear section 102 of the injection device 1. It will be understood that the rear section 102 of the injection device 1 is receivable through the opening 309 so that it extends through the aperture 306.

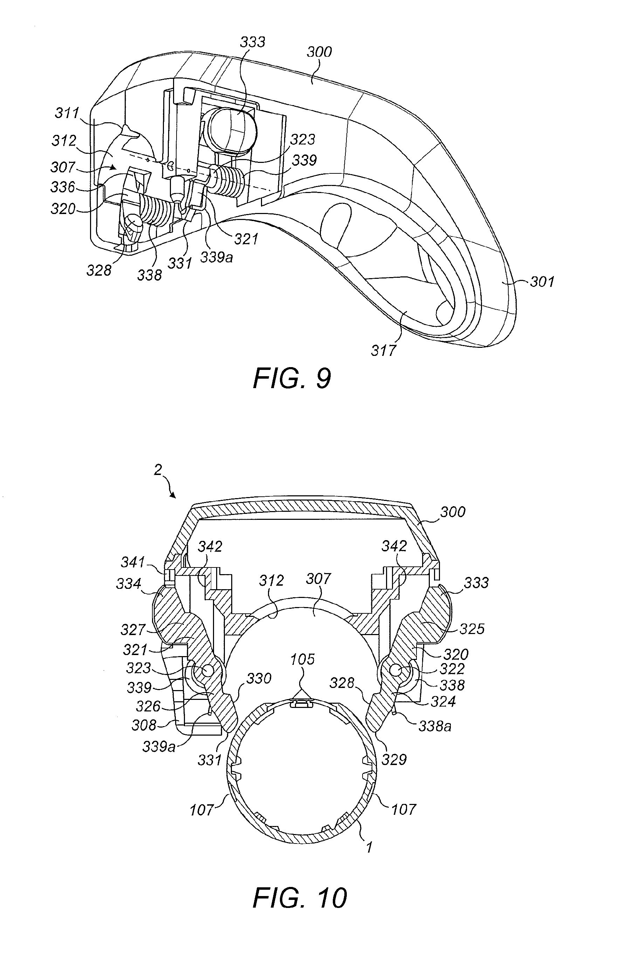

The channel 307 is shown in Figures 9 and 10. The channel 307 has a base 312. The base 312 of the channel 307 is arcuate in cross-section. The base 312 extends parallel to the longitudinal axis of the body 300. The shape of the base 312 corresponds to the outer surface of the rear section 102 of the injection device 1. Therefore, the rear section 102 of the injection device is receivable therein and the outer surface of the medicament delivery device 1 locates against the channel base 312. The optical sensor 25 may be embedded in the channel base 312 to face into the channel 312.

The collar 301 defines an upper part 314 and a lower part 315. The upper part 314 is integrally formed with the body 300 and thus extends from the base 312 of the channel 307. The lower part 315 opposes, but is at least partially offset from, the upper part 314. In the present embodiment, the upper part 314 is defined by the upper half of the inner surface of the collar 301 and the lower part 315 is defined by the lower half of the inner surface of the collar 301. The inner surface of the collar 301 defines a cylinder, with the base 312 of the channel 307 extending from the cylindrical surface. Therefore, the arcuate base of the channel 307 and the inner cylindrical surface of the collar are formed to arc about the same longitudinal axis.

A lower locating surface 317 is defined on the lower part 315 of the collar 301. An upper locating surface 316 is defined on the upper part 314 of the collar 301. The upper and lower locating surfaces 316, 317 oppose each other. When the injection device is received through the aperture, the upper and lower locating surfaces 316, 317 are configured to locate against the outer surface of the medicament delivery device 1. The locating surfaces 316, 317 are brought into contact with the medicament delivery device 1 by rotating the data collection device 2 about an axis extending perpendicular to the major axis of the opening 309 so that the upper and lower locating surfaces are moved towards the outer surface of the medicament delivery device 1. The central axis of the cylindrical aperture 306 extending through the collar 301 is brought into co-axial alignment with the longitudinal axis of the medicament delivery device 1.

Although, in the present embodiment, the collar 301 has a cylindrical arrangement, it will be understood that a break may be formed in the collar 301 to form two collar portions (not shown) with distal ends that distend towards each other, but are spaced from each other, so that the collar portions define a passage. A section of one or both of the collar portions defines the lower locating surface 317. Similarly, in an alternative arrangement an arcuate collar member (not shown) may extend from one side of the channel to define a channel and the lower locating surface 317.

In the present embodiment, the base 312 of the channel extends co-planar with the upper part of the collar 301. Therefore, the base 312 of the channel 307 also locates against the outer surface of the medicament delivery device 1 when the data collection device 2 is rotated about an axis extending perpendicular to the major axis of the opening 309 so that the upper and lower locating surfaces 316, 317 are moved to lie against the outer surface of the medicament delivery device 1. Therefore, it will be understood that the upper locating surface may be formed by the upper part of the collar 301, or by the base 312 of the channel 307. Alternatively, the lower locating surface 317 is formed on one or more locating elements protruding into the aperture from the lower part of the collar 301. Similarly, the upper locating surface 316 may be formed on one or more locating elements protruding into the aperture 306 or the channel 307.

A rib receiving recess 311 is formed in the base 312 of the channel 307. The rib receiving recess 311 is dimensioned to receive the rib 105 protruding from the outer surface 106 of the injection device 1. The rib receiving recess 311 is dimensioned so as to correspond closely to the shape and size of the locating rib 105 that is present on the medicament delivery device 1. The rib receiving recess 311 is slightly larger than the locating rib 105 so as to ensure that the locating rib 105 can be easily located within the recess 311. Therefore, the rib receiving recess 311 acts as an alignment element for locating the body in a specific position relative to the outer surface 106 of the medicament delivery device 1 when the rib 105 is received in the rib receiving recess 311. The rib receiving recess 311 therefore aids the correct alignment and orientation of the body 300 on the medicament delivery device 1.

The rib receiving recess 311 is formed at the end of the data collection device 2 that is closest to the dosage knob 12 when the data collection device 2 is fitted to the injection device 1.

As described above, the data collection device 2 comprises the housing 20 and the mating arrangement. The mating arrangement is configured to embrace the housing 10 of medicament delivery device 1 so that data collection device 2 sits tightly on housing 10 of medicament delivery device 1, but is nevertheless removable from the medicament delivery device 1, for instance when the medicament delivery device 1 has to be replaced.

A first engaging arm 320 is pivotably mounted in the body 300 of the data collection device 2. A second engaging arm 321 is pivotably mounted in the body 300 of the data collection device 2. The first and second engaging arms 320, 321 are disposed on opposing sides of the body 300.

The first and second engaging arms 320, 321 extend from the channel 307. The first and second engaging arms 320, 321 extend from opposing sides of the channel 307 and are spaced from each other. The first and second engaging arms 320, 321 are disposed so that the data collection device 2 is receivable therebetween.

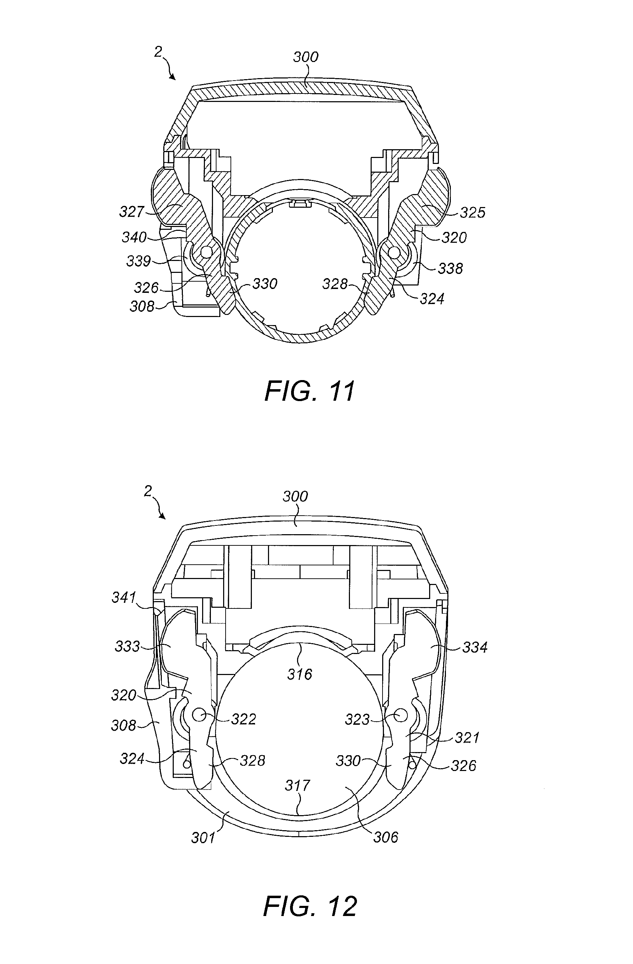

Wings 308 depend from each side of the body 300. In Figures 11 to 14 one of the wings 308 is omitted. The wings 308 act as protective walls. Each wing 308 protrudes from the lower side 303 of the body 300. The body 300 has two opposing wings 308 which depend from opposing sides of the channel 307. The wings 308 may be integrally formed with the body 300. The wings 308 extend the side walls of the channel 307. In the present embodiment, the wings 308 extend over the mating elements, that is engaging arms 320, 321. Therefore, the wings 308 act as protective elements or cover portions.

As noted above, at least part of one or both wings 308 provides a non-opaque section, so that at least part of the mating arrangement of the medicament delivery device 1 can be viewed through part, or all, of the wings 308. For example, part or all of the wings 308 may be formed of a translucent, or a transparent, material. In this particular example, the wings 308 include a section 350 that is non-opaque.

The first and second engaging arms 320, 321 are elongate members. The first engaging arm 320 is pivotably mounted to the body 300 of the data collection device 2 by a first pivot axle 322. The second engaging arm 321 is pivotably mounted to the body 300 of the data collection device 2 by a second pivot axle 323. The first pivot axle 322 is a cylindrical shaft protruding from opposing sides of the first engaging arm 320. The second pivot axle 323 is a cylindrical shaft protruding from opposing sides of the second engaging arm 321. The first and second pivot axles 322, 323 extend perpendicular to the longitudinal axes of the first and second engaging arms 320, 321 respectively.

An engaging part 324 of the first engaging arm 320 extends from the first pivot axle 322. A release part 325 of the first engaging arm 320 extends from the opposing side of the first pivot axle 322. The engaging part 324 and release part 325 together form the first engaging arm 320. The first pivot axle 322 is disposed at a midsection of the first engaging arm 320.

An engaging part 326 of the second engaging arm 321 extends from the second pivot axle 323. A release part 327 of the second engaging arm 321 extends from the opposing side of the second pivot axle 323. The engaging part 326 and release part 327 together form the second engaging arm 321. The second pivot axle 323 is disposed at a midsection of the second engaging arm 321.

In the present embodiment, the first and second pivot axles 322, 323 are fixedly mounted to the respective first and second engaging arm 320, 321 so that each arm 320, 321 and corresponding pivot axle 322, 323 rotate together in the body 300. In such an arrangement, the opposing ends of the pivot axles 322, 323 are received in receiving holes in the body 300 in which the ends are able to rotate. Therefore, the pivot axles 322, 323 and engaging arms 320, 321 are able to pivot in the body 300. In such an embodiment the pivot axles 322, 323 are fixedly mounted to the engaging arms 320, 321. However, in an alternative embodiment the first and second engaging arms 320, 321 are rotatable about their respective pivot axles 322, 323. In such an arrangement, the pivot axles 322, 323 may be fixedly mounted to the body 300.

The first engaging arm 320 has a first protuberance 328 disposed at a free end 329 of the engaging part 324 of the first engaging arm 320. The second engaging arm 321 has a second protuberance 330 disposed at a free end 331 of the engaging part 324 of the second engaging arm 321. Each protuberance 328, 330 acts as an engaging element to engage in the indents 107, 108 formed in the outer surface of the rear section 102 of the medicament delivery device 1. The first protuberance 328 on the first engaging arm 320 is configured to be received in the left indent 107. The second protuberance 330 on the second engaging arm 321 is configured to be received in the right indent 108. The protuberances 328, 330 are shaped to correspond to the shapes of the indents 107, 108 respectively. In this way, the protuberances 328, 330 fit within the corresponding indents 107, 108 respectively when the data collection device 2 is correctly positioned on the medicament delivery device 1. The external dimensions of the protuberances 328, 330 are slightly smaller than the internal dimensions of the indents 107, 108 so as to ensure that the protuberances 322 fit within their respective indent. Therefore, indents 107, 108 act as engaging portions.

Since at least part of one or both wings 308 is a non-opaque section 350, a user may view part of the mating arrangement located underneath the wings 308 to verify visually that the first and second protuberances 328, 330 of the first and second engaging arms 320, 321 are correctly engaged with the first and second indents 107, 108 on the housing 10 of the medicament delivery device 1 and, therefore, that the data collection device 2 is firmly attached to the medicament delivery device 1. This allows an additional check on the correct positioning and alignment of the data collection device 2 when mounted on the medicament delivery device 1, to facilitate reliable determination and recording of medicament dosage information.

As noted above, the provision of the non-opaque section 350 may also facilitate the aligning of the data collection device 2 with the medicament delivery device 1 because the user can view the features of the data collection device 2 and the medicament delivery device 1 that are to be brought into engagement and can more easily determine the direction in which the data collecting device 2 should be moved relative to the medicament delivery device 1 in order to achieve the desired positioning and alignment. Without the ability to view such features, the user would have to shift and rotate the data collection device 2 relative to the medicament delivery device 1 blindly in a try-and-error mode until a firm connection is established, which the user might detect by feeling that the two devices have been coupled together, for example because it becomes more difficult to move the data collection device 2 relative to the medicament delivery device 1, and/or by a click sound produced when the mating arrangement engages the first and second indents 107, 108 and/or the rib 105 is received in the rib receiving recess 311. The provision of the non-opaque or transparent section 350 provides the ability to view such features, therefore the user sees the directions where to shift and/or rotate the data collection device 2 relative to the medicament delivery device 1 until a firm connection is established. In addition to the visual confirmation, the firm connection can be detected also by feeling that the two devices have been coupled together and/or by a click sound, as explained before.

Medical devices intended for operation by a patient are often designed to have a clean appearance in which technological features, such as sensors, engaging members and so on, tend to be concealed. This is intended to avoid intimidating the patient and improve patient compliance. However, the provision of a non-opaque section 350 can facilitate the placement of the data collection device 2, and improve reliability of the data collection performed by the data collection device 2. By making the data collection device 2 easier to position correctly, a patient may be less likely to avoid using the data collection device 2, even though the visual appearance of the data collection device 2 reveals certain technological features to the user.

Moreover, the provision of a non-opaque section 350 can focus the attention of the user on the relevant part of the mating arrangement and the cooperating formation, that is first and/or second indents 107, 108, on the medicament delivery device 1, and limit their view of other technological features of the data collection device 2 and medicament delivery device 1, since many of the other parts of the data collection device 2 and medicament delivery device 1 remain obscured. In particular, where the non-opaque section 350 is configured as a window in an opaque housing, the user's attention may be focussed in this manner very effectively.

In the present embodiment, the first protuberance 328 is shaped to correspond closely to the shape of the left indent 107, as shown in Figure 13. In this way, the first protuberance 328 fits snugly within the left indent 107 when the data collection device 2 is correctly positioned on the medicament delivery device 1. The second protuberance 330 is shaped similarly to the first protuberance 328, although the width of the second protuberance 330 is smaller width than the width of the first protuberance 328. In the present arrangement, this is achieved with an "almond" shaped geometry, as shown in Figure 14. This is the reason for the end face of the first protuberance 328 having a larger area than the second protuberance 330. The different sizes for the protuberances 328,330 helps the protuberances find engagement within the indents 107. The first protuberance 328 can be considered to be a master to the second protuberance 330, which is a slave. However, it will be understood that in an alternative embodiment the protuberances 328, 330 are identical.

Although the engaging elements on the first and second engaging arms 320, 321 are formed by protuberances which are configured to be received in corresponding indents 107 on the medicament delivery device 1, it will be understood that alternative arrangements are envisaged. For example, in an alternative arrangement, the engaging element on one or both of the engaging elements 320, 321 is formed by a notch or indent formed proximate to the free end 329, 330 of the or each engaging element 320, 321 which is configured to locate over and/or receive a corresponding protrusion or ledge on the medicament delivery device 1 so that the engaging arms 320, 321 engage with the medicament delivery device 1.

The release part 325 of the first engaging arm 320 has a first actuating section 333 at its distal end to the pivot axle 322. The first actuating section 333 is disposed on the opposing side of the first engaging arm 320 to the first protuberance 328. The first actuating section 333 is disposed at an opposing end of the first engaging arm 320 to the first protuberance 328.

The release part 327 of the second engaging arm 321 has a second actuating section 334 at its distal end to the second pivot axle 323. The second actuating section 334 is disposed on the opposing side of the second engaging arm 321 to the second protuberance 330. The second actuating section 334 is disposed at an opposing end of the second engaging arm 321 to the second protuberance 330.

The actuating sections 333, 334 are formed by upstanding portions or stepped portions of the engaging arms 320, 322. Each actuating section 333, 334 has an outer face against which a user is able to act, as will become apparent hereinafter. The actuating sections 333, 334 are configured to extend through the body 300 of the data collection device 2 on opposing sides of the body 300 so that they are exposed on an outer side. Therefore, a user is able to urge the engaging arms 320, 322 to pivot.

The first engaging arm 320 is received in a one side of the body 300. The second engaging arm 321 is received in another side of the body 300. The engaging arms 320, 321 are disposed behind the wings 308 that depend from the body 300. The wings 308 may be formed from a transparent material. This allows a user to be able to view the locations of the engaging arms 320, 321 relative to the indents 107, which may help the user to locate the data collection device 2 correctly on the medicament delivery device 1. As can be seen from Figures 9 to 12, the wings 308 extend slightly further in a downwards direction than the engaging arms 320, 321. The engaging arms 320, 322 are rotatably mounted in the body 300 by the pivot axles 322, 323.

The first engaging arm 320 is received in a first arm receiving space 335 on one side of the body 300 and the second engaging arm 321 is received in an arm receiving space 335 on one side of the body 300. The engaging parts 324, 326 of the engaging arms 320 extend through gaps 336 in the channel base 312 so that they face into the channel 307 from opposing sides of the channel base 312. This is the engaging parts 324, 326 of the engaging arms 320 are able to protrude into the channel 307 from the gaps 336.

A first torsion spring 338, acting as a resilient member, acts on the first engaging arm 320. The first torsion spring 338 acts on the first engaging arm 320 to urge the first engaging arm 320 to rotate. The first torsion spring 338 biases the engaging part 324 of the first engaging arm 320 to protrude into the channel 307. In the present embodiment, the first torsion spring 338 acts on the engaging part 324 to bias the first engaging arm 320, however it will be understood that the first torsion spring 338 may act on the release part 325.

A second torsion spring 339, acting as a resilient member, acts on the second engaging arm 321. The second torsion spring 339 acts on the second engaging arm 321 to urge the second engaging arm 321 to rotate. The second torsion spring 339 biases the engaging part 326 of the second engaging arm 321 to protrude into the channel 307. In the present embodiment, the second torsion spring 339 acts on the engaging part 326 to bias the second engaging arm 321, however it will be understood that, in another embodiment, the second torsion spring 339 may act on the release part 326.

The first and second torsion springs 338, 339 are disposed on the first and second pivot axles 322, 323 respectively. Therefore, a compact arrangement may be achieved. One end of each torsion spring 338, 339 acts on the body 300 of the data collection device 2 and the other end acts on the corresponding engaging arm 320, 321. The end of each torsion spring 338, 339 acting on the engaging arms 320, 321 has a protruding leg 338a, 339a which acts on a rear side of the corresponding engaging arm 320, 321. The protruding legs 338a, 339a of each torsion spring 338, 339 are received in a corresponding notch 343 on the rear side of the corresponding engaging arm 320, 321. In an alternative embodiment, one end of the torsion springs 338, 339 acts on the pivot axles 322, 323 to cause the engaging arms 320, 321 to rotate.

In the present arrangement, each torsion spring 338a, 339a is configured to impart an urging force on the corresponding engaging arm in the range of 2N to 10N. Preferably, the urging force imparted on each engaging arm is in the range of 3N to 7N, and more preferably in the range of 4N to 5N. However, it will be understood that the urging force imparted by each engaging arm is dependent on the relationship of the engaging arm and the requirements regarding attachment force and detachment force.

Referring to Figures 11 and 15, the central axis defining the rotational axis of each torsion spring 338a, 339a is offset from the rotational axis of the corresponding pivot axles 322, 323, and therefore the rotational axis of the engaging arms 320, 321. Eccentric members are mounted on the pivot axles to eccentrically mount the torsion springs around the pivot axles. For example, in Figure 15 an eccentric member 323a is mounted on the second pivot axle 323. The second pivot axle 323 is disposed proximate to a circumferential edge of the eccentric member 323a. The second torsion spring 338b extends around the eccentric member 323a. Although, in the present embodiment, the eccentric members are mounted to the corresponding pivot axle, it will be understood that in an alternative embodiment the or each eccentric member may be integrally formed with the corresponding pivot axle. The positioning of the rotational axis of the torsion springs 338a, 339a offset from the rotational axis of the pivot axles ensures that the angle under which the protuberances 328,330 are urged into the indents 107 is kept flat so that the holding force in a radial direction is maximised.

The engaging parts 324, 326 of the engaging arms 320, 321 are splayed towards each other. The effect of the resilience of the torsion springs 338, 339 is to bias the engaging part 324, 326 of each arm 320 into a certain position. The position into which the engaging part 324, 326 of each engaging arm 320, 321 is a disengaged position is shown in Figure 10, wherein they are initially located such that the distance between the innermost surfaces of the protuberances 328, 330 at the free end 329, 331 of the arms 320, 321 is slightly less than the distance between the bottoms of the indents 107. The effect of the bias of the torsion springs 338, 339 of each arm 320, 321 is to resist movement of the protuberances 328, 330 and the engaging parts 324, 326 of the arms 320, 321 away from one another. End stops 340 are formed in the body 300 to limit rotation of the engaging arms 320, 321. The arms 320, 321 act against the end stops 340 when the torsion springs 338, 339 bias the arms into their unengaged position, as shown in Figure 10. In the present embodiment, the end stops 340 are formed by the wings 308.

The engaging arms 320, 321, acting as support members, are restrained from moving in a direction along the longitudinal axis of the elongate body 300. This assists in maintaining the data collection device 2 in the correct position after engagement of the data collection device on the medicament delivery pen 1 even in the presence of forces acting to move the data collection device 2 along the longitudinal axis of the medicament delivery device 1.

The actuating sections 333, 334 of the engaging arms form left and right buttons extending from opposing sides of the body 300. An aperture 341 is formed through each side of the body 300 through which the actuating sections 333, 334 of the engaging arms extend to protrude from the body 300 when the engaging arms 320, 321 are biased into their disengaged position, as shown in Figure 10.

Although, in the present embodiment, the data collection device 2 has first and second engaging arms 320, 321, it will be understood that in an alternative embodiment, the data collection device 2 might have one engaging arm only. Alternatively, the data collection device 2 may have a pivotable first engaging arm, and a second fixed engaging arm. An advantage of having two pivotable engaging arms is that they help ensure a correct engagement.

When the actuating sections 333, 334 are pressed inwardly by a user the engaging arms 320, 321 are urged to rotate about the pivot axles defining pivot axes of the engaging arms 320, 321. The arms 320, 321 act against the bias of the torsion springs 338, 339 so that the engaging parts 324, 326 move outwardly.

When a user applies a force to the actuating sections 333, 334 of the arms 320, 321, the arms 320, 321 pivot about their axis and the actuating sections 333, 334 move inwardly. The engaging parts 324, 326 of the two arms 320, 321 are urged to rotate about their respective pivot axles 322, 323. Therefore, the free ends 329, 331 of the arm engaging parts 324, 326 are urged away from each other into a detached position. The release of the pressing force on each actuating section 333, 334 releases the biasing force acting on each arm 320, 321 and so the engaging part of each arm is urged to return to its neutral position due to the resilience of the torsion springs 338, 339.

As is shown in Figure 6, the data collection device 2 is initially located with respect to the medicament delivery device 1 such that the opening 309 to the aperture 306 in the collar 301 is aligned with the rear end of the medicament delivery device 1. The body 300 is orientated so that the longitudinal axis of the medicament delivery device receiving channel 307 is inclined with respect to the longitudinal axis of the medicament delivery device 1.

The collar 301 is then slid over the rear section 102 of the medicament delivery device 1, as shown in Figure 7. The width of the front opening 309 at its minor axis or conjugate diameter corresponds to or is slightly greater than the diameter of the rear section 102 of the injection device 1. The width of the front opening 309 at its major axis or transverse diameter is greater than the diameter of the rear section 102 of the injection device 1. Therefore, the rear section 102 of the injection device 1 is received through the aperture 306 of the collar 301.

In order to locate the data collection device 2 on the medicament delivery device 1, the data collection device 2 is rotated relative to the medicament delivery device 1 about an axis extending perpendicular to the major axis of the opening 309. The longitudinal axes of the medicament delivery device 1 and the 307 are rotated towards each other. Furthermore, the upper and lower locating surfaces 316, 317 are moved towards the outer surface of the medicament delivery device 1.

As the data collection device 2 and the medicament delivery device 1 are rotated relative to each other the free ends 329, 331 of the first and second engaging arms 320, 321, in particular the protuberances 328, 330, are brought into contact with the outer surface of the housing 10 of the medicament delivery device 1. The protuberances 328, 330 here contact the housing to the left and right sides of the dosage window 13.

In order to engage the data collection device 2 with the medicament delivery device 1, the user first arranges the data collection device 2 with respect to the medicament delivery device 1 as shown in Figure 7, and then applies a further force downwards on the data collection device 2 while at the same time applying a force upwards on the medicament delivery device 1. Therefore, the data collection device 2 and injection device 1 are urged to rotate relative to each other about the collar 301. A biasing force is therefore applied to the protuberances 328, 330 by the outer surface of the rear section 102 of the medicament delivery device 1. As the medicament delivery device 1 and the data collection device 2 are urged to move closer together, the biasing force results in the arms being urged away from each other. The arms 320, 321 are urged to deflect outwardly, and to rotate about their pivot axes against the action of the torsion springs 338, 339. The torsion springs 338, 339 cause a reaction force to be applied on each arm due to the resilience of the torsion springs 338, 339, which resists entry of the medicament delivery device 1 into the channel 307. However, as the data collection device 2 is further rotated over the medicament delivery device 1, the protuberances 328, 330 become aligned with the left and right indent 107 and, due to the resilience of the torsion springs 338, 339, engage with the indents 107, as shown in Figure 13. The pivot axles help to ensure the correct alignment of the arms 320, 321 and therefore the protuberances with the indents 107.

As the data collection device 2 engages the protuberances 328, 330 in the indents 107, the rear section 2 of the medicament delivery device 1 is received in the channel 207. The lower locating surface 317 of the collar 301 is urged into contact with a lower side of the outer surface of the medicament delivery device 1, and the upper locating surface 316 is urged into contact with an opposing side of the outer surface of the medicament delivery device 1. Once the protuberances 328, 330 engage in the indents 107, there is significant resistance to further movement of the data collection device 2 relative to the medicament delivery device 1, due in part to the lower and upper locating surfaces 316, 317 abutting the outer surface of the medicament delivery device. The upper and lower locating surfaces 316, 317 are partially offset from each other, with the upper locating surface 317 being disposed between the lower locating surface and the protuberances 328, 330. The action of the torsion springs 338, 339 acts to urge the medicament delivery device 1 against the base 312 of the channel 307. Movement of the data collection device 2 relative to the medicament delivery device 1 in the opposite direction is restricted by the action of the torsion springs 338, 339 and due to the protuberances 328, 330 being engaged in the indents 107.

It will be understood that the body 300 of the data collection device 2 is mated to the medicament delivery device 1 by the collar 301 extending circumferentially around the medicament delivery device 1 at a front end of the body 300, and the protuberances 328, 330 engaging in the indents 107 at the rear end of the body 300.

In the event that the user places the data collection device 2 onto the medicament delivery device 1 at a location such that the data collection device 2 is rotated slightly about its longitudinal axis relative to its desired position, the rib 105 will not be received in the rib receiving recess 311 in the body 300. In this case, the data collection device 2 is prevented from being located fully over the medicament delivery device 1 by the rib 105 resting against the base 312 of the channel 307, spaced from the correct location within the rib receiving recess 311. Furthermore, the protuberances 328, 330 will be urged against the outer surface of the rear section 102 of the medicament delivery device 1, but will not locate in the corresponding indents 107. A user would know that the data collection device 2 had not mated correctly with the medicament delivery device 1 because they would not have received any haptic feedback from the mating of the protuberances 328, 330 with the indents 107. They would also notice that the rear end of the data collection device was separated from the injection device 1. To correctly locate the data collection device 2 in position with respect to the medicament delivery device 1, a user can simply rotate the data collection device 2 relative to the medicament delivery device 1 about the longitudinal axis of the medicament delivery device 1. As the data collection device 2 and the medicament delivery device 1 move relative to one another, the locating rib and the rib receiving recess 311 become aligned with each other. At this point the protuberances 328, 330 also align with the indents 107 and engage therewith. Haptic feedback is therefore provided by the mating of the protuberances 328, 330 with the indents 107 and the user can determine that the data collection device 2 and medicament delivery device 1 are properly positioned with respect to one another.

Similarly, if the data collection device 2 is not correctly aligned with the medicament delivery device 1 in a longitudinal direction, the rib 105 and rib receiving recess 311 will not be aligned and the rib will not locate in the recess 311. Furthermore, the protuberances 328, 330 will be offset from the indents 107. Relative movement of the data collection device 2 and the medicament delivery device 1 in a direction along the longitudinal axis of the medicament delivery device 1 will cause the rib to locate in the recess 311 and the protuberances 328, 330 to locate in the indents 107. At this point, the data collection device 2 and the medicament delivery device 1 are fully engaged with one another.

Once the protuberances 328, 330 are engaged in the indents 107, the medicament delivery device 1 is fully located within channel 307 as shown in Figures 8 and 11. Here, the outermost surface of the dosage window 13 is aligned with a lowermost surface of the upper part of the data collection device 2. The data collection device 2 is shaped such that the medicament delivery device 1 fits snugly within the channel 307 and there are multiple points or areas of contact between the exterior surface of the housing 10 of the medicament delivery device 1 and the lowermost surface of the data collection device 2 when the data collection device 2 and the medicament delivery device 1 are in this relative position. When the data collection device 2 is located with respect to the medicament delivery device 1 such that the protuberances 322 are located within the indents 107 respectively, the rib 105 is engaged within the rib receiving recess 311. Correct alignment of the data collection device 2 with respect to the medicament delivery device 1 is thus provided in two ways: firstly, by the location of the rib 118 within the rib receiving recess 311 and secondly by the locating of the protuberances 328, 330 within the indents 107.

It will be appreciated that the above arrangement prevents movement of the medicament delivery device 1 relative to the data collection device 2. Movement of the data collection device 2 in a radial direction from the medicament delivery device 1 is prevented by the collar 301 extending around the medicament delivery device 1. Movement of the data collection device 2 in a radial direction from the medicament delivery device 1 is also prevented by the protuberances 328, 330 engaging in the indents 107. The engagement of the protuberances 328, 330 in the indents 107 also prevents movement of the data collection device 2 along the longitudinal axis of the medicament delivery device 1 and about the longitudinal axis of the medicament delivery device 1. Additionally, movement of the data collection device 2 about the longitudinal axis of the medicament delivery device and along the longitudinal axis of the medicament delivery device 1 is further prevented by the rib 105 being received in the rib receiving recess 311.

In order to remove the data collection device 2 from the medicament delivery device 1, a user exerts a pressing force on the actuating sections 333, 334 of the engaging arms 320, 321 protruding from the outside of the body 300. The pressing force may be exerted by a finger and thumb. As the two actuating sections 333, 334 are depressed by the pressing force exerted by the user, engaging arms 320, 321 are urged to rotate about their axes.

Referring to Figure 16, the actuating sections 333, 334 are configured to extend through the body 300 of the data collection device 2 on opposing sides of the body 300 so that they are exposed on an outer side. The wings 308 are adjacent the actuating sections 333, 334. An outer surface 345 of each wing 308 protrudes from a body outer surface 346 and an edge 347 is defined along the outer surface 345 of each wing 308. The edge 347 forms a step. The actuating sections 333, 334 protrude from the body outer surface 346. However, the outer face of the relevant actuating section 333, 334 does not protrude from the outer surface 345 of the wing 308. That is, the outer face of the relevant actuating section 333, 334 lies planar with the outer surface 345 of the corresponding wing 308. An edge 348 of the corresponding actuating section 333, 334 extends from the edge 347 defined along the outer surface 345 of each wing 308. Therefore, accidental pressing or movement of the actuating sections 333, 334 is prevented by protruding design surfaces of the wings 308 which cover the button to the rear and the bottom sides.

The arms 320, 321 are urged to pivot about their pivot axles 322, 323. Therefore, the protuberances 328, 330 on each arm 320 are urged apart. This causes the protuberances 328, 330 to disengage from the indents 107. Therefore, the engaging arms 320, 321 move from their engaged position to their detached position. Rotation of the arms 320, 321 is limited by ends faces 342 against which the arms 320, 321 locate.

Once the protuberances 328, 330 have been urged from the indents 107, the user may then rotate the rear end of the data collection device 2 away from the medicament delivery device 1 about an axis extending perpendicular to the major axis of the opening 309. As the protuberances 328, 330 are free of the indents 107, the user experiences relatively little resistance as the rear end of the body 300 is moved away from the medicament delivery device.

It will be understood that the cooperation between the rib 105 and the rib receiving recess 311 does not present any obstacle to separating the data collection device 2 and the medicament delivery device 1 in this way. The medicament delivery device 1 may then be slid from the aperture 306 formed in the collar 301.

Although in the above embodiment, the lower locating surface is defined by the lower part of the inner surface of the collar 301, it will be understood that the lower locating surface may be formed by another element on the lower part of the collar 301. Furthermore, a break may be formed in the collar.

Although in the above described embodiment the upper locating surface is defined by the upper part of the inner surface of the collar 301, it will be understood that the upper locating portion may be defined by the base of the channel 307. In such an arrangement, the inner surface of the collar 301 and the base of the channel 307 may be stepped from each other. The upper locating portion may be formed by another element on the upper part of the collar 301. Alternatively, the upper locating portion may be formed by another element on the base of channel 307.

Although the rib 105 and rib receiving recess aid orientation and alignment of the data collection device 2 on the medicament delivery device 1 in the above described embodiments, it will be appreciated that in an alternative arrangement the rib 105 and rib receiving recess are omitted and the correct alignment between the data collection device 2 and the medicament delivery device 1 is provided by mating of the protuberances and the indents 107.

Other alternative arrangements for ensuring a correct relative position between the data collection device 2 and the medicament delivery device 1 will be envisaged by the skilled person, and all such alternatives are within the scope of the invention except when explicitly excluded by the language of the claims.

## Claims

1. A data collection device comprising:
a body; and
a mating arrangement configured to releasably attach the body to a medicament delivery device, the mating arrangement having at least one engaging member configured to engage with a cooperating formation on the medicament delivery device; and
a cover portion that overlies at least part of said engaging member, at least a part of the cover portion being a non-opaque section through which said at least part of said engaging member is viewable.

2. A data collection device according to claim 1, wherein the mating arrangement may include one or more resilient members arranged to bias the at least one engaging member into engagement with the cooperating formation when the data collection device is attached to the medicament delivery device.

3. A data collection device according to claim 2, comprising an actuating section configured to urge the at least one engaging member out of engagement with the cooperating formation when the data collection device is attached to the medicament delivery device.

4. A data collection device according to claim 2 or 3, wherein:
the at least one engaging member is a first engaging arm;
the mating arrangement further comprises a second engaging arm; and
said one or more resilient members bias free ends of the first and second engaging arms towards each other.

5. A data collection device according any of the preceding claims, wherein the at least one engaging member is rotatable between an engaged position and a detached position.

6. A data collection device according to any preceding claim, wherein the mating arrangement further comprises a collar which is configured to receive a medicament delivery device.

7. A data collection device according to claim 6, wherein the collar is configured to be pivotable between a first position in which the medicament delivery device is slidably receivable through the collar and a secured position in which the at least one engaging member is engageable with the cooperating formation to attach the data collection device to the medicament delivery device.

8. A data collection device according to any of the preceding claims, comprising:
a camera unit configured to capture images of a display on the medicament dosage indicator of the medicament delivery device when the data collection device is attached to the medicament delivery device.

9. A data collection device according to any of claims 1 to 7, comprising:
a sensing arrangement comprising one or more sensors arranged to detect movement of a dosage programming element of the medicament delivery device.

10. A data collection device according to claim 8 or claim 9, comprising a processing arrangement configured to determine a medicament dosage amount programmed into the medicament delivery device based on at least one image of the medicament dosage indicator captured by said camera unit or on movement detected by said sensing arrangement.

11. A data collection device according to any preceding claim, wherein the non-opaque section comprises a window provided in said cover portion.

12. A data collection device according to claim 11 wherein the window has an optical power to provide a magnified view of said at least part of said engaging member.

13. A data collection device according to claim 11 or 12, wherein the window is made from glass.

14. A data collection device according to any of claims 1 - 10, wherein the non-opaque section comprises a sub-section that has an optical power to provide a magnified view of said at least part of said engaging member.

15. A medicament delivery system comprising:
a medicament delivery device; and
a data collection device according to any preceding claim.
